# EUROPÄISCHE PATENTSCHRIFT

(11) **EP 1 413 323 B1**
(45) Veröffentlichungstag und Bekanntmachung des Hinweises auf die Patenterteilung: **13.10.2010**
(21) Anmeldenummer: 03090350.4
(22) Anmeldetag: 16.10.2003
(51) Int. Cl.: A61L 27/12, C03C 3/076, C04B 35/447

(54) **Glas als Sinterhilfsmittel und offenporiger Formkörper sowie Verfahren zu seiner Herstellung**
Glass as sintering aid, open-pore shaped article and method for its production
Verre comme aide de frittage, corps à pores ouverts et méthode de sa production

(30) Priorität: 21.10.2002 DE 10249626; 21.10.2002 DE 10261992
(43) Veröffentlichungstag der Anmeldung: 28.04.2004
(73) Patentinhaber: BAM Bundesanstalt für Materialforschung und -prüfung, 12205 Berlin (DE)
(72) Erfinder: Berger, Georg, 16341 Zepernick (DE); Gildenhaar, Renate, 13086 Berlin (DE); Spitzer, Andrea, 10827 Berlin (DE)
(74) Vertreter: Walter, Wolf-Jürgen

(56) Entgegenhaltungen:
- EP-A- 0 405 556
- EP-A- 0 885 856
- US-A- 4 135 935
- US-A- 4 308 064
- US-A- 4 708 652

## Beschreibung

Die Erfindung betrifft ein Glas als Sinterhilfsmittel für einen resorbierbaren calciumphosphathaltigen Formkörper sowie ein Herstellungsverfahren für diesen Formkörper.

Die anorganischen Knochenersatzmaterialien lassen sich in resorbierbare und langzeitstabile Werkstoffe gliedern. Ihre Auswahl und Einsatz richten sich nach der spezifischen medizinischen Indikation. Stand der Technik beim Einsatz resorbierbarer, den direkten Knochenkontakt fördernder Produkte sind Werkstoffe auf der Basis von Tricalciumphosphaten. Überwiegend eingesetzt werden Granulate mit unterschiedlichen Korngrößen. Problematisch dabei ist es, dass im Verlaufe der Biodegradation und des parallel verlaufenden Knochenwachstums die verbleibenden Granulatpartikel im operierten und mit dem Granulat aufgefüllten Knochendefekt zusammengepresst werden und die restlose Auflösung verhindern. Dieses Problem kann durch verschiedene Ansätze gelöst werden. Eine Methode besteht darin schneller resorbierbare Werkstoffe zu entwickeln (z.B. EP 541564 B1 und US-A-4308064), eine weitere besteht darin die Granulate mit innerer Porosität zu versehen und damit die Auflösung nach ihrer Zusammenpressung zu befördern (z.B. DE 19744809 C1).

Prinzipiell lässt sich das Problem auch dadurch lösen, dass man einen offen- und gleichzeitig großporigen Formkörper herstellt. Dies kann durch Pressen/isostatisches Pressen des Ausgangsmaterials und anschließendem Versintern sowie Einbringen der Porenstruktur durch Bohren etc. mit verschiedensten Techniken (mechanisch, mit Trockeneis etc.), durch sogenannte Free Form Fabrication-Techniken oder nach der bekannten Schwammimprägniertechnik mit nachfolgendem Sintern (Schwartzwalder-Somers-Verfahren) erfolgen.

Der Nachteil dieser Lösungen wird vielfach dabei sein, dass das zu verwendende Tricalciumphosphat nicht genügend versintert und somit die Formkörper mechanisch relativ instabil sind.

Der Erfindung liegt die Aufgabe zugrunde, die Sinterfähigkeit von calciumphosphathaltigen Werkstoffen entscheidend zu verbessern und gleichzeitig die Resorbierbarkeit und Biokompatibilität des damit hergestellten Formkörpers zu erhalten bzw. zu verbessern.

Erfindungsgemäß bereitgestellt wird daher ein Glas als Sinterhilfsmittel für einen resorbierbaren calciumphosphathaltigen Werkstoff, wobei der Werkstoff β-Tricalciumphosphat ist und das Glas eine chemische Zusammensetzung von 68-78 Gew-% SiO₂, 5-12 Gew-% MgO und 12-27 Gew-% Na₂O hat EP-885 856-A2 beschreibt die Verwendung dieser Glaszusammensetzung als Sinterhilfsmittel für Apatit-Glaskeramik.

Besonders vorteilhaft hat das Glas eine chemische Zusammensetzung von 73-78 Gew-% SiO₂, 8-11 Gew-% MgO und 12-19 Gew-% Na₂O, insbesondere von 74-75 Gew-% SiO₂, 8,5-10 Gew-% MgO und 14,5-17 Gew-% Na₂O.

Das Glas hat einen Anteil von 0,5-15 Gew-% neben 85-99,5 Gew-% Tricalciumphosphat (TCP).

Die Komponenten TCP und Glas als amorphe Komponente werden zunächst separat hergestellt und separat aufgemahlen, sodann vermischt, ggf. wiederum in einer Mühle, daraus ein Schlicker mit weiteren Zuatzstoffen hergestellt und beispielsweise auf einen Pol-yurethan(PUR)-Schwamm aufgebracht. Nach dem Trocknen wird dieser Schwamm einer Temperaturbehandlung ausgesetzt, wobei der Schwamm rückstandslos verbrennt und ein Formkörper erhalten wird, der röntgendiffraktographisch lediglich die Kristallphase TCP enthält.

Damit ergeben sich Formkörper, die jeweils maximal folgende Einzelkomponenten gemäß der Synthesezusammensetzung in Gew.-% enthalten:

| | |
|---|---|
| CaO | 53,97 |
| P₂O₅ | 45,53 |
| SiO₂ | 11,40 |
| Na₂O | 4,05 |
| MgO | 1,8 |

Die Komponenten werden dabei so eingestellt, dass maximal
100 % der Synthesezusammensetzung erreicht werden.

Überraschenderweise wurde nun gefunden, dass diese zuvor beschriebene 2-Komponenten-Variante zu dem gewünschten Sinterprodukt führt, während eine sofortige Zusammenführung aller Komponenten den Sinterprozess nicht in der gewünschten Weise unterstützt. Ohne Zusatz der getrennt hergestellten amorphen Glasphase erhält man beim Sintern des verschlickerten TCP auf einem Polyurethanschwamm keine feste Struktur, sondern es tritt teilweise ein Abbröckeln des Sinterproduktes auf.

Die Erfindung betrifft daher auch ein Verfahren zur Herstellung eines resorbierbaren calciumphosphathaltigen Formkörpers, dadurch gekennzeichnet, dass man ein Glas, bestehend aus 68-78 Gew-% SiO₂, 5-12 Gew-% MgO und 12-27 Gew-% Na₂O schmilzt, es auf eine Korngröße D₅₀ von 0,7-2 µm bringt, es mit einem β-Tricalciumphosphat der Korngröße D₅₀ von 1-7,5 µm vermischt, und es in üblicher Weise in eine gewünschte Form überführt und den Formkörper durch Sintern bei 1150 bis 1350°C herstellt, mit der Maßgabe, dass die Korngröße des β-TCP nicht kleiner ist als die des Glases.

Gegenstand der Erfindung ist somit auch ein offenporiger Formkörper auf der Basis von β-Tricalciumphosphat, dadurch gekennzeichnet, dass er folgende Zusammensetzung aufweist (in Gew-%) 46,1 bis 54,0 CaO, 38,9 bis 45,5 P₂O₅, 0,005 bis 11,4 SiO₂, 0,001 bis 4,05 Na₂O und 0,0005 bis 1,8 MgO und röntgenographisch die kristalline Phase β-Tricalciumphosphat aufweist. Die Reinheitsanforderungen für β-TCP entsprechen der Norm ASTM F 1088-87 (re-approved 1992).

Ein weiterer Gegenstand der Erfindung ist der oben genannte offenporige Formkörper, hergestellt durch separate Herstellung von β-Tricalciumphosphat und eines Glases aus 68-78 Gew-% SiO₂, 5-12 Gew-% MgO und 12-27 Gew-% Na₂O, und dem Vermischen von 99,5-85 Gew-% β-Tricalciumphosphat und 0,5-15 Gew-% Glas, und in üblicher Weise Verschlickern des Gemisches, Auftragen auf ein offenporiges Gerüst, vorzugsweise ein offenporiges PUR-Gerüst, und Sintern des Formkörpers bei 1150 bis 1350 °C.

Ein bevorzugter Anteil Glas als Zusatzstoff liegt im Bereich 1-10 Gew-%, vorteilhaft 4-8 %, insbesondere 5-7 Gew-%.

Die Erfindung ist auf dieses Anwendungsbeispiel keinesfalls begrenzt, sondern es können auch weitere in dem o.g. Zusammensetzungsfeld befindliche Schmelzprodukte hergestellt werden, die zu analogem Ergebnis führen.

Wesentlich für die Wirkung ist dabei, dass die amorphe Komponente fein aufgemahlen wird, z.B. in einer Rührwerksmühle, und der D₅₀-Wert vorzugsweise unter dem des Tricalciumphosphates liegt und in jedem Fall aber deutlich unter 2 µm liegen sollte.

Die eingesetzten amorphen Schmelzprodukte zeichnen sich dadurch aus, dass sie chemisch relativ unbeständig sind, was die Resorbierbarkeit des Gesamtkörpers fördert. Die Biokompatibilität ist dadurch gegeben, dass an sich nur physiologische Bestandteile in dem Sinterhilfsmittel enthalten sind. Kritisch wäre allein der etwas höhere Silicium-Gehalt zu bewerten, da an sich im Blut bzw. Knochengewebe nur geringfügige Gehalte an Silicium vorkommen. Jedoch bezogen auf den Gesamtkörper ist der Zusatz insgesamt betrachtet ebenfalls sehr gering. In jüngster Zeit vermittelte Eindrücke aus der Fachliteratur zeigen auf, dass eine - im Gegensatz zum hier angewendeten 2-Komponenten-Verfahren - innige Vermischung aller Ausgangsmaterialien mit Silicium die Resorbierbarkeit durch Stimulation der Osteoclasten (knochenabbauende Zellen) begünstigen soll. Dies wurde an Werkstoffen-Gemischen aus Si-α-TCP und Hydroxylapatit mit offener Porosität bestimmt, die durch die Verwendung von kolloidalem SiO₂-Solen hergestellt wurden (Langstaff, S. et al.: Resorbable bioceramics based on stabilized CP. Part I: Rational design, sample preparation and materials characterization, Biomaterials 20 (1999) 1727-1741; Part II: Evaluation of biological response, Biomaterials 22 (2001) 135-150).

Die Herstellung kann dabei nach dem bereits eingangs genannten Schwartzwalder-Somers-Verfahren erfolgen, bei dem ein Schliker auf einen PUR-Schwamm aufgetragen wird, und anschließend der Schwamm ausgebrannt wird.

Ein weiteres Verfahren, auf das die erfindungsgemäße Herstellung mit Glas/β-TCP vorteilhaft angewandt werden kann, ist die Free Form Fabrication (oder Rapid Prototyping). Bei diesem Verfahren erfolgt normalerweise ein Lasersintern von TCP mit oder ohne Polymere als Zuschlagstoffe, wobei die Polymeren ebenfalls später ausgebrannt werden. Im vorliegenden Fall wird zusätzlich das Glas als Sinterhilfsmittel eingebracht.

Die Erfindung soll nachstehend durch Beispiele näher erläutert werden. Alle Angaben erfolgen in Gewichtsprozent, sofern nichts anderes angegeben ist.

### Beispiel 1

Es wird beta-TCP nach in der Literatur hinreichend beschriebenen Verfahren hergestellt. Dieses ß-TCP wird aufgemahlen, so dass ein sinterfähiges Pulver mit einem D₅₀-Partikeldurchmesser von ca. 1,7 µm entsteht.

Es wird ein Schmelzprodukt hergestellt mit folgender Zusammensetzung (in Gew-%) SiO₂: 74,97; MgO: 9,22 und Na₂O: 15,81 (eingeschmolzen als 27,04 Na₂CO₃). Das Produkt wird geschmolzen und aufgemahlen, so dass das Pulver mit einem D₅₀-Partikeldurchmesser von 1,23 µm vorliegt.

Von dem hergestellten β-TCP werden 94 Gew-% und von dem Schmelzprodukt werden 6 Gew-% zu einem Feststoffgemisch vermengt, 41,68 g eines Isopropanol-Wasser-Gemisches (30:70) werden mit 0,57 g Dispergator C64 und 57,75 g des Feststoffgemisches versetzt und 3 Minuten in der Planetenmühle zu einem Schlicker mit cremiger Konsistenz vermischt. Dieser Schlicker wird auf PUR-Schwämme mit offener Porosität mit 80 bis 20 ppi (Poren pro Zoll) durch mehrmaliges Eintauchen und Ausdrücken aufgebracht, an der Luft getrocknet, im Trockenschrank bei etwa 100C 2 Stunden getrocknet und dann langsam mit 100°C pro Stunde auf 1300°C erhitzt und 6 Stunden gehalten. Im Ergebnis liegt ein spongiosa-artiges Produkt mit einem, dem Ausgangsschwamm ähnlichen Aufbau vor, und der PUR-Schwamm ist rückstandslos ausgebrannt.

Das β-TCP als einzige röntgenographisch feststellbare Kristallphase zeigt eine sehr gute Stabilität entsprechend der vorgegebenen Schwammstruktur; es tritt kein Abbröckeln in der Struktur auf, und der Formkörper zeigt eine Resorbierbarkeit, die der von nach bekannten Verfahren hergestelltem ß-TCP entspricht.

### Beispiel 2 (Vergleichsbeispiel)

Es wird ein β-TCP auf übliche Weise hergestellt und nach der Schwammimprägniertechnik auf einen PUR-Schwamm aufgetragen und bei 1300C in gleicher Weise wie im Beispiel 1 gesintert. Der gesinterte Formkörper zeigt nach dem Abkühlen an mehreren Stellen deutliche Abbröckelerscheinungen, die sich bei mechanischem Kontakt mit ihm fortsetzen.

### Beispiel 3

Es wird wie im Beispiel 1 verfahren, jedoch folgende Glaszusammensetzung verwendet.
SiO₂ 71,5 %; MgO 9,5 %; Na₂O 19,0 %.
Diese Glaszusammensetzung wurde geschmolzen, gefrittet und aufgemahlen, so dass ein Pulver mit einem Partikeldurchmesser D₅₀ von 1,43 vorliegt.

### Beispiel 4

Es wird ein Glas nach Beispiel 1 hergestellt. Von diesem Glas werden 9 % und vom β-TCP 91 % eingesetzt. 30 g dieses Pulvergemisches werden mit 30 ml einer 5-%igen wässrigen Polyethylenglycol-Lösung (MG: 2000) unter Zugabe von 750 µl Isopropylalkohol zu einem Schlicker verarbeitet, wobei zur Erhöhung des Feststoffgehaltes im Schlicker 0,3 % eines Dispergators (CE 64, Fa. Schimmer und Schwartz) eingesetzt werden. Zur besseren Vermischung wird dieses Gemisch im Homogenisator (16000 U/min; Fa. Heidolph) 2 Minuten behandelt.

## Patentansprüche

1. Verwendung von Glas als Sinterhilfsmittel für einen resorbierbaren calciumphosphathaltigen Werkstoff, **dadurch gekennzeichnet, dass** der Werkstoff β-Tricalciumphosphat ist und das Glas eine chemische Zusammensetzung von 68-78 Gew-% SiO₂, 5-12 Gew-% MgO und 12-27 Gew-% Na₂O hat.

2. Verwendung nach Anspruch 1, **dadurch gekennzeichnet, dass** das Glas eine chemische Zusammensetzung von 73-78 Gew-% SiO₂, 8-11 Gew-% MgO und 12-19 Gew-% Na₂O hat.

3. Verwendung nach Anspruch 1, **dadurch gekennzeichnet, dass** das Glas eine chemische Zusammensetzung von 74-75 Gew-% SiO₂, 8,5-10 Gew-% MgO und 14,5-17 Gew-% Na₂O hat.

4. Verwendung nach Anspruch 1, **dadurch gekennzeichnet, dass** das Glas einen Anteil von 0,5-15 Gew-% hat neben 85-99,5 Gew-% Tricalciumphosphat.

5. Verwendung nach Anspruch 4, **dadurch gekennzeichnet, dass** es einen Anteil von 4-8 Gew-% hat.

6. Verfahren zur Herstellung eines resorbierbaren calciumphosphathaltigen Formkörpers, **dadurch gekennzeichnet, dass** man ein Glas, bestehend aus 68-78 Gew-% SiO₂, 5-12 Gew-% MgO und 12-27 Gew-% Na₂O schmilzt, es auf eine Korngröße D₅₀ von 0,7-2 µm bringt, es mit einem β-Tricalciumphosphat der Korngröße D₅₀ von 1-7,5 µm vermischt, und es in eine gewünschte Form überführt und den Formkörper durch Sintern bei 1150 bis 1350°C und anschließendes Abkühlen herstellt, mit der Maßgabe, dass die Korngröße des β-Tricalciumphosphats nicht kleiner ist als die des Glases.

7. Verfahren nach Anspruch 6, **dadurch gekennzeichnet, dass** die Formgebung nach dem Schwartzwalder-Somers-Verfahren oder dem Free Form Fabrication-Verfahren erfolgt.

8. Offenporiger Formkörper auf der Basis von β-Tricalciumphosphat, **dadurch gekennzeichnet, dass** er folgende Zusammensetzung aufweist (in Gew-%) 46,1 bis 54,0 CaO, 38,9 bis 45,5 P₂O₅, 0,005 bis 11,4 SiO₂, 0,001 bis 4,05 Na₂O und 0,0005 bis 1,8 MgO und röntgenographisch allein die kristalline Phase β-Tricalciumphosphat aufweist, hergestellt durch separate Herstellung von β-Tricalciumphosphat und separate Herstellung eines Glases aus 68-78 Gew-% SiO₂, 5-12 Gew-% MgO und 12-27 Gew-% Na₂O, und durch Vermischen von 99,5-85 Gew-% β-Tricalciumphosphat und 0,5-15 Gew-% Glas, und in üblicher Weise Verschlickern des Gemisches, Auftragen auf ein offenporiges Gerüst und Sintern des Formkörpers bei 1150 bis 1350°C und anschließendes Abkühlen, mit der Maßgabe, dass die Korngröße des β-TCP 1-7,5 µm beträgt, die Korngröße des Glases 0,7-2 µm beträgt und die Korngröße des β-TCP nicht kleiner ist als die des Glases.

## Claims

1. Use of a glass as a sintering aid for a resorbable material comprising calcium phosphate, **characterized in that** the material is β-tricalcium phosphate and the glass has a chemical composition of 68-78% by weight SiO₂, 5-12% by weight MgO and 12-27% by weight Na₂O.

2. Use according to Claim 1, wherein said glass has a chemical composition of 73-78% by weight SiO₂, 8-11% by weight MgO and 12-19% by weight Na₂O.

3. Use according to Claim 1, wherein said glass has a chemical composition of 74-75% by weight SiO₂, 8.5-10% by weight MgO and 14.5-17% by weight Na₂O.

4. Use according to Claim 1, wherein said glass makes up 0.5-15% by weight while tricalcium phosphate makes up 85-99.5% by weight.

5. Use according to Claim 4, wherein said glass makes up 4-8% by weight.

6. A method for manufacturing a resorbable moulded body comprising calcium phosphate, **characterized in that** a glass consisting of 68-78% by weight SiO₂, 5-12% by weight MgO and 12-27% by weight Na₂O is melted, ground until a grain size D₅₀ of 0.7-2µm is achieved and mixed with β-tricalcium phosphate having a grain size D₅₀ of 1-7.5µm, the mixture is given the desired shape and the moulded body is produced by sintering said mixture at between 1,150 and 1,350°C and subsequently cooling it, with the provisio that the grain size of β-TCP must not be smaller than that of the glass.

7. A method according to Claim 6, wherein shaping is carried out using the Schwartzwalder-Somers process or the free-form fabrication method.

8. An open-pore moulded body based on β-tricalcium phosphate (β-TCP), **characterized in that** said moulded body has a composition ranging between (in % by weight) 46.1 and 54.0 CaO, 38.9 and 45.5 P₂O₅, 0.005 and 11.4 SiO₂, 0.001 and 4.05 Na₂O and 0.0005 and 1.8 MgO and solely comprises β-tricalcium phosphate as a crystalline phase according to roentgenographic analyses and is manufactured by separately producing β-tricalcium phosphate and separately producing a glass consisting of 68-78% by weight SiO₂, 5-12% by weight MgO and 12-27% by weight Na₂O, mixing 99.5-85% by weight β-tricalcium phosphate and 0.5-15% by weight glass, processing the mixture into a slurry in a usual manner, applying it onto an open-pore sponge and sintering it at between 1,150 and 1,350°C to obtain after cooling the moulded body, with the provisio that the grain size of β-TCP is 1-7.5 µm, the grain size of the glass is 0.7-2 µm and the grain size of β-TCP must not be smaller than that of the glass.

## Revendications

1. Utilisation de verre comme aide de frittage pour un matériau en phosphate de calcium résorbable, **caractérisé en ce que** le matériau est le β-phosphate tricalcique, et **en ce que** le verre a la composition chimique consistant en 68-78 % en poids de SiO₂, 5-12 % en poids de MgO et 12-27 % en poids de Na₂O.

2. Utilisation selon la revendication 1, **caractérisée en ce que** le verre a la composition chimique consistant en 73-78 % en poids de SiO₂, 8-11 % en poids de MgO et 12-19 % en poids de Na₂O.

3. Utilisation selon la revendication 1, **caractérisée en ce que** le verre a la composition chimique consistant en 74-75 % en poids de SiO₂, en poids de MgO et 14,5-17 % en poids de Na₂O.

4. Utilisation selon la revendication 1, **caractérisée en ce que** le verre est présent en une proportion de 0,5-15 % en poids, en plus des 85-99,5 % en poids de phosphate tricalcique.

5. Utilisation selon la revendication 4, **caractérisée en ce qu'**il est présent en une proportion de 4-8 % en poids.

6. Procédé de préparation d'un corps moulé en phosphate de calcium résorbable, **caractérisé en ce que** l'on fait fondre un verre, consistant en 68-78 % en poids de SiO₂, 5-12 % en poids de MgO et 12-27 % en poids de Na₂O, il est porté à une granulométrie D₅₀ de 0,7-2 µm, il est mélangé à un β-phosphate tricalcique de granulométrie D₅₀ de 1-7,5 µm, et il est converti en une forme souhaitée et le corps moulé est préparé par frittage à une température allant de 1150 à 1350°C et ensuite, refroidissement, avec la condition de la granulométrie du β-phosphate tricalcique n'est pas inférieure à celle du verre.

7. Procédé selon la revendication 6, **caractérisé en ce que** le moulage est réalisé selon le procédé de Schwartzwalder-Somers ou le procédé Free Form Fabrication.

8. Corps moulé à pores ouverts à base de β-phosphate tricalcique, **caractérisé en ce qu'**il présente la composition suivante (en % en poids) 46,1 à 54,0 CaO, 38,9 à 45,5 P₂O₅, 0,005 à 11,4 SiO₂, 0,001 à 4,05 Na₂O et 0,0005 à 1,8 MgO et il présente par diffraction des rayons X, seulement la phase cristalline β-phosphate tricalcique, préparé par préparation séparée du β-phosphate tricalcique et préparation séparée d'un verre consistant en 68-78 % en poids de SiO₂, 5-12 % en poids de MgO et 12-27 % en poids de Na₂O, et par mélange de 99,5-85 % en poids de β-phosphate tricalcique et de 0,5-15 % en poids de verre et formation d'une suspension du mélange de manière usuelle, application sur une structure à pores ouverts et frittage du corps moulé à des températures allant de 1150 à 1350°C et ensuite, refroidissement, avec la condition que la granulométrie du β-phosphate tricalcique se situe à 1-7,5 µm, la granulométrie du verre se situe à 0,7-2 µm et la granulométrie du β-phosphate tricalcique n'est pas inférieure à celle du verre.
